(19) Europäisches Patentamt / European Patent Office / Office européen des brevets

(11) **EP 3 330 971 A1**

(12) **EUROPEAN PATENT APPLICATION**

(43) Date of publication:
06.06.2018 Bulletin 2018/23

(51) Int Cl.:
*G16H 20/30* (2018.01)

(21) Application number: 17205498.3

(22) Date of filing: 05.12.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: 05.12.2016 AU 2016905002

(71) Applicant: **Impact Technologies Australia Pty Ltd 6160 Fremantle, Western Australia (AU)**

(72) Inventors:
• **AUSTIN, David**
  **Sydney, New South Wales 2000 (AU)**
• **VEGAR, Michael**
  **Sydney, New South Wales 2000 (AU)**
• **LANG, Lucas**
  **Sydney, New South Wales 2000 (AU)**

(74) Representative: **Brann AB**
**P.O. Box 3690**
**Drottninggatan 27**
**103 59 Stockholm (SE)**

(54) **METHODS, DEVICES AND TECHNOLOGY FRAMEWORKS CONFIGURED TO ENABLE REAL-TIME MONITORING OF HEAD IMPACT DATA FOR PARTICIPANTS IN CONTACT SPORTS**

(57) Technology disclosed herein relates to monitoring of effects relevant to head impacts and/or injuries in humans. Embodiments of the invention have been particularly developed for enabling real-time monitoring of head impact data for participants in contact sports systems. This finds application, for example, in the context of managing brain injury risks in the context of sporting activities.

**FIG. 1**

**Description**

**FIELD OF THE INVENTION**

The present invention relates to methods, devices and technology frameworks configured to enable monitoring of effects relevant to head impacts and/or injuries in humans. Embodiments of the invention have been particularly developed for enabling real-time monitoring of head impact data for participants in contact sports systems. While some embodiments will be described herein with particular reference to those applications, it will be appreciated that inventions disclosed is not limited to such fields of use, and is applicable in broader contexts.

**BACKGROUND**

Any discussion of the background art throughout the specification should in no way be considered as an admission that such art is widely known or forms part of common general knowledge in the field.

In the course of various activities (such as sports), participants' heads experience accelerations and decelerations (negative accelerations), including from head impacts. These accelerations can lead to brain injury, and even death. A single significant brain injury can present immediate symptoms and is classified as a concussion. Potentially more concerning is the cumulative effect of a series of smaller (or sub-concussive) impacts. This cumulative effect has been recently discovered in American football players and is called chronic traumatic encephalopahy (CTE). Initial CTE symptoms include lack of attention, disorientation, dizziness and headaches. Later stage CTE symptoms include social instability, erratic behaviour, dementia, impeded speech and deafness. Currently, CTE can only be diagnosed by (post-mortem) direct tissue analysis. Players from boxing, American football, soccer, rugby, wrestling, ice hockey, mixed martial arts, Australian rules football, baseball, lacrosse and other contact sports are believed to be at risk of CTE.

It is becoming increasingly common to monitor participants during sporting activities via sensor devices. However, sensor systems for use in sport must be compact and unobtrusive. This leads to challenges in the contest of data management: for example, a sensor-enabled device with three accelerometers and three gyroscopes, each running at 400Hz, may generate over one thousand bytes of data per impact event.

For accurate detection of head impacts via a motion-sensitive sensor, the sensor must be rigidly attached to the head. In this context, attempts have been made to make use of such sensor devices in the context of monitoring head impacts, including mouthguard impact sensors (see, for example, US 8,104,324 and US 2012/0143526). In many ways, mouthguards are ideal for sports sensors. However, operations within the space of the mouthguard are particularly challenging. Space is extremely limited, requiring small circuit board areas and severely limiting the battery size and power. Space and battery constraints in turn limit the amount of computation that can be carried out inside the mouthguard. For example, performing the complex calculations to rotate and translate the impact data to the centre of the brain is difficult within the mouthguard. Thus, for accurate impact detection, the full set of impact data must be communicated to a bigger and more capable computation device.

The present inventors have recognised a further challenge for mouthguard sensors is that communications from inside the mouth are difficult. When the mouth is closed, the flesh of the lips, cheeks and head absorbs most of the transmitted radio power. In experiments conducted by the present inventors, the radio signal was attenuated by approximately 50dB with the mouth closed and the range reduced from 10m to 2m. The physical size of the mouthguard also limits the possible size of in-mouthguard radio antennae, which in turn limits the antenna gain available. As a result, it is challenging to achieve long-range, reliable communications from a mouthguard sensing device. The teachings of US 2100/0181420 propose handling communications for a mouthguard sensor via a mesh network. However, a mesh network for mouthguard sensing devices involved practical challenges, chiefly because of the limited range of in-mouth communication devices. For example, with 11-18 players on a field of between 50 metres to 110 metres wide, the average player-to-player spacing will from 4.5 metres (assuming players lined up across the field - e.g. American football) to 25 metres (Australian football players spread evenly over field). Clearly, worst-case spacing will be much higher. For low power devices, these distances are simply too high and a reliable mesh network cannot be formed.

**SUMMARY OF THE INVENTION**

It is an object of the present invention to overcome or ameliorate at least one of the disadvantages of the prior art, or to provide a useful alternative.

One embodiment provides a system configured to enable analysis of human head impacts, the system including: one or more human-worn hardware sets, wherein each human-worn hardware set includes: (i) a mouthguard having one or more sensors, wherein the sensors are configured to collectively provide a primary motion data signal, a processor configured to receive the primary motion data signal, and a communications module that is configured to wirelessly transmit, via a first wireless communications protocol, a secondary motion data signal derived from the primary motion data signal; (ii) a secondary transmitter device, wherein the secondary transmitter device is configured to receive the secondary motion data signal via the first wireless communications protocol, and in response communicate, via a second wireless communications protocol, a tertiary motion data signal derived from the secondary motion data signal; and a computer system that is configured to receive respective tertiary motion data signals from the or each of the one or more human-worn hardware sets, wherein the computer system is configured to process tertiary motion data thereby to determine estimated head impact data for users associated with the or each of the one or more human-worn hardware sets.

One embodiment provides a system wherein the first wireless communications protocol is Bluetooth Low Energy (BLE).

One embodiment provides a system wherein the secondary transmitter device is provided via one of: a helmet; a garment; and another form of body-worn device.

One embodiment provides a system wherein the secondary transmitter device is configured to receive an input signal via the second wireless communications protocol.

One embodiment provides a system wherein the secondary transmitter device is configured to cause delivery of an alert signal a wearer of the human worn hardware set.

One embodiment provides a system wherein the alert signal is delivered by the secondary transmitter device.

One embodiment provides a system wherein the alert signal is delivered by the mouthguard.

One embodiment provides a system wherein the signal is delivered via one of: haptic feedback; bone conduction; or a visible means.

One embodiment provides a system wherein the input signal is defined by the computer system.

One embodiment provides a system wherein the computer system is configured to process the tertiary motion data signal thereby to: (i) perform an impact analysis process; and (ii) in response to the impact analysis process, selectively cause delivery the input signal to the secondary transmitter device.

One embodiment provides a system wherein the secondary transmitter device performs compression-based processing of the secondary motion data signal as part of defining the tertiary motion data signal.

One embodiment provides a system wherein the secondary transmitter device performs is configured to process the primary motion data signal thereby to: (i) perform an impact analysis process; and (ii) in response to the impact analysis process" selectively cause delivery of an alert signal a wearer of the human worn hardware set.

One embodiment provides a system wherein the alert signal is delivered by the secondary transmitter device.

One embodiment provides a system wherein the alert signal is delivered by the mouthguard.

One embodiment provides a system wherein the signal is delivered via one of: haptic feedback; bone conduction; or a visible means.

One embodiment provides a system wherein at least one of the mouthguards includes at least seven accelerometer sensors mounted on the body; and a processing device mounted on the body, wherein the processing device is configured to receive motion data from the at least seven accelerometer sensors.

One embodiment provides a system wherein determine estimated head impact data includes: receiving motion data derived from the least seven accelerometer sensors of a given one of the mouthguards via a tertiary motion data signal; and processing the motion data via an optimisation method thereby to, based on a combination of data derived from each of the at least seven accelerometer sensors, determining values representative of both linear and rotational accelerations of a brain of the human head.

One embodiment provides a system wherein at least one of the mouthguards includes one or more of: an accelerometer; a gyroscope; a temperature sensor; a heart rate sensor, a step sensor, and a saliva composition sensor.

One embodiment provides a system wherein two or more of the primary motion data signal, secondary motion data signal and tertiary motion data signal include substantially identical data.

One embodiment provides a system wherein two or more of the primary motion data signal, secondary motion data signal and tertiary motion data signal are substantially identical.

One embodiment provides a system wherein the first wireless communication protocol and the second wireless communication protocol are defined by a common form of wireless communications protocol.

One embodiment provides a secondary transmitter device configured to operate in a system as described herein.

One embodiment provides a system configured to enable analysis of human activity, the system including: one or more human-worn hardware sets, wherein each human-worn hardware set includes: (i) a mouthguard having one or more sensors, wherein the sensors are configured to collectively provide a primary data signal, a processor configured to receive the primary data signal, and a communications module that is configured to wirelessly transmit, via a first wireless communications protocol, a secondary data signal derived from the primary data signal; (ii) a secondary transmitter device, wherein the secondary transmitter device is configured to receive the secondary data signal via the first wireless communications protocol, and in response communicate, via a second wireless communications protocol, a tertiary data signal derived from the secondary data signal; and a computer system that is configured to receive respective tertiary data signals from the or each of the one or more human-worn hardware sets, wherein the computer system is configured to process tertiary data thereby to determine human activity assessment data for users associated with the or each of the one or more human-worn hardware sets.

One embodiment provides a computer program product for performing a method as described herein.

One embodiment provides a non-transitive carrier medium for carrying computer executable code that, when executed on a processor, causes the processor to perform a method as described herein.

One embodiment provides a system configured for performing a method as described herein.

Reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

As used herein, the term "exemplary" is used in the sense of providing examples, as opposed to indicating quality. That is, an "exemplary embodiment" is an embodiment provided as an example, as opposed to necessarily being an embodiment of exemplary quality.

## BRIEF DESCRIPTION OF THE DRAWINGS

Embodiments of the invention will now be described, by way of example only, with reference to the accompanying drawings in which:

FIG. 1 schematically illustrates a system according to one embodiment, including an example mouthguard device.

FIG. 2A illustrates a mouthguard device according to one embodiment.

FIG. 2B illustrates components of a mouthguard device according to one embodiment.

FIG. 2C illustrates a mouthguard device according to one embodiment.

FIG. 3A illustrates a method according to one embodiment.

FIG. 3B illustrates a method according to one embodiment.

FIG. 3C illustrates a method according to one embodiment.

FIG. 3D illustrates a method according to one embodiment.

FIG. 4A illustrates typical impact accelerations.

FIG. 4B illustrates an impact activity function according to one embodiment.

FIG. 5A illustrates an example secondary transmitter device.

FIG. 5B illustrates an example secondary transmitter device.

**DETAILED DESCRIPTION**

Described herein are methods, devices and technology framework configured to enable monitoring of effects relevant to head impacts and/or injuries in humans. Embodiments of the invention have been particularly developed for enabling real-time monitoring of head impact data for participants in contact sports systems. Also described is technology related more specifically to sensor-enabled mouthguards. Embodiments of the invention have been particularly developed to provide sensor-enabled mouthguards configured to enable monitoring of head impact data via multiple accelerometers, and data processing methods configured to analyse data derived from multiple accelerometers carried by a sensor-enabled mouthguard. While some embodiments will be described herein with particular reference to those applications, it will be appreciated that inventions disclosed is not limited to such fields of use, and is applicable in broader contexts.

The technology is described primarily by reference to applications in the context of monitoring participants in team sporting activities that occur on a playing field, such as various codes of football. However, it will be appreciated that the technology is equally applicable to a range of other environments where head impacts and the like are a potential concern.

*Example Framework*

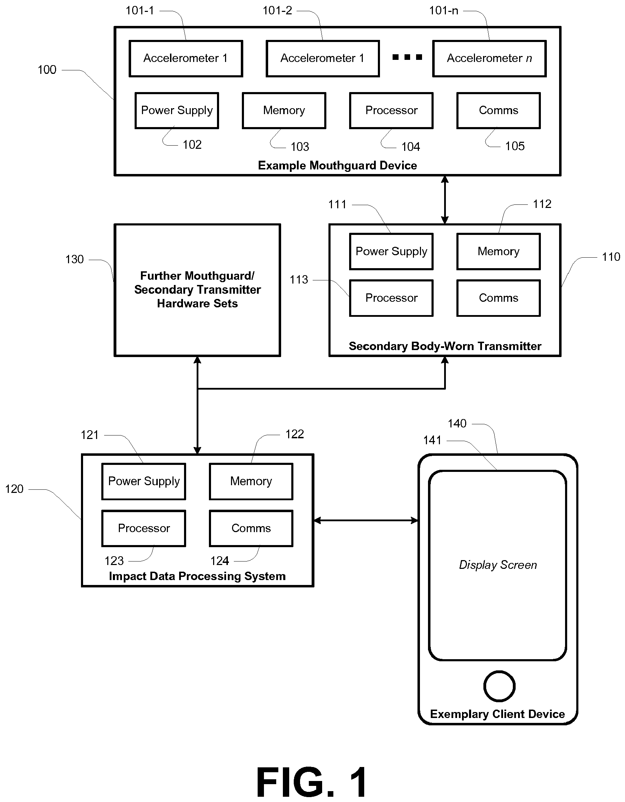

FIG. 1 illustrates a framework according to one embodiment, in the form of a system configured to enable analysis of human head impacts. It should be appreciated that further embodiments substitute various components shown herein for alternate components that perform corresponding functionalities. For example, the mouthguard device shown is an example only, and various other forms of mouthguard may be used. Furthermore, the technology is not limited to analysis of human head impacts, and may be adapted to other forms of activity monitoring (for example mouthgards with sensors configured to monitor steps, heart rate, and so on).

The framework of FIG. 1 has been specifically designed to manage technical problems associated with monitoring of participants via mouthguard carried sensors. In particular, there are technical challenges in terms of both communication ranges and data processing, which are managed by aspects f the technology described below.

The framework includes one or more human-worn hardware sets (for example in one implementation the technology is applied to monitor a single individual participant in an activity, whereas in other implementations the technology is applied to monitor multiple participants, such as an entire team in a team-based activity). Each human-worn hardware set includes: (i) a mouthguard device (such as device 101) having one or more sensors; and (ii) a secondary transmitter device (such as 110).

Each mouthguard device includes one or more sensors that are configured to collectively provide a primary motion data signal. These sensors may include any one or more of accelerometers, gyroscopes, temperature sensors, saliva composition sensors, and other forms of sensor (for example including heart rate and/or step sensors). FIG. 1 illustrates an example mouthguard device 100 which includes a plurality of accelerometers (101-1 to 101-n). This example is not intended to be limiting, although various embodiments are described in detail further below in relation to particular mouthguard and processing technology based on a mouthguard device including seven or more accelerometer sensors (for example three spaced apart 3-axis accelerometer devices). Each mouthguard also includes a processor configured to receive the primary motion data signal, and a communications module that is configured to wirelessly transmit, via a first wireless communications protocol, a secondary motion data signal derived from the primary motion data signal. As illustrated, example mouthguard device 100 includes a processor 104 and a communications module 105. Mouthguard device 100 additionally includes a power supply 102, and a memory module 103 (which carries computer-executable software instructions that are executed via processor 104 thereby to enable the performance of computer-implemented methods).

The processing involved in converting the primary motion data signal to the secondary motion data signal varies between embodiments. For example, in some embodiments raw data from sensor components is subjected to minimal processing other than packetizing for wireless transmission. In further embodiments the mouthguard's on-board processor is configured to perform various forms of compression, pre-processing and/or buffering. It will be appreciated that this is a matter of design choice based, at least in part, on selection of the processing device (for example in terms of processing power).

The first wireless communications protocol is preferably a low-power short-range protocol. For example, preferred embodiments make use of Bluetooth Low Energy (BLE). BLE was identified as a particularly useful technology due to the ability to miniaturise power and transmission components into a form suitable for embedding in a mouthguard wholly contained in a human mouth (including an antenna device). However, it has an inherently limited range.

To account for the limited range of BLE (and in further embodiments limited ranges of other primary communications protocols), each secondary transmitter device is configured to receive the secondary motion data signal via the first wireless communications protocol, and in response communicate, via a second wireless communications protocol, a tertiary motion data signal derived from the secondary motion data signal. The secondary wireless communications protocol is preferably reliable over a medium-range (for example 20-200m). In some embodiments communications protocols such as WiFi and Bluetooth are used. However, in other embodiments other wireless communications protocols (including custom radio frequency protocols) are used. The secondary transmitter device is less constrained by size, along for a larger power supply and antenna given that it is not constrained by the internal space in a participant's mouth. Preferably, the secondary transmitter device is a body-worn device, such as GPS tracker, helmet mounted device, heart rate monitor, watch, phone or the like. Substantially any electronic device having one or more radios for receiving data from the mouthguard and transmitting via an appropriate form of second wireless communications protocol.

The processing involved in converting the secondary motion data signal to the tertiary motion data signal varies between embodiments. Given potential to incorporate additional processing power in the body-worn device (for example where a smartphone or similarly powerful device is used), there are advantages associated with performing a degree of data simplification, for example data compression. This reduces the amount of data required to be transferred via the secondary communications protocol. Other forms of processing include filtering, artefact identification (for example identifying data attributes potentially representative of an impact event thereby to reduce the need for ongoing transmission of irrelevant data), so on. In some embodiments the processing includes classification, for example to classify observed events (e.g. predicted impacts) into one or more of a plurality of predefined categories. For instance, these may include categories defined for common forms of head impacts (frontal, side, etc), and optionally include sport-specific impact types (for example types of impacts associated with particular sports such as boxing, MMA and the like). In some embodiments processing is performed thereby to perform either or both (i) a comprehensive impact analysis (for example as discussed below), and (ii) a basic impact analysis (for example to identify data values above predefined thresholds for alerts to be generated).

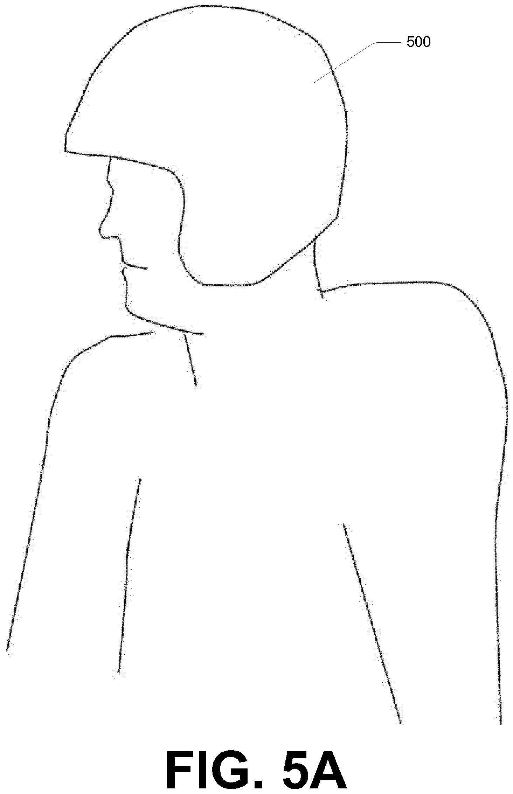

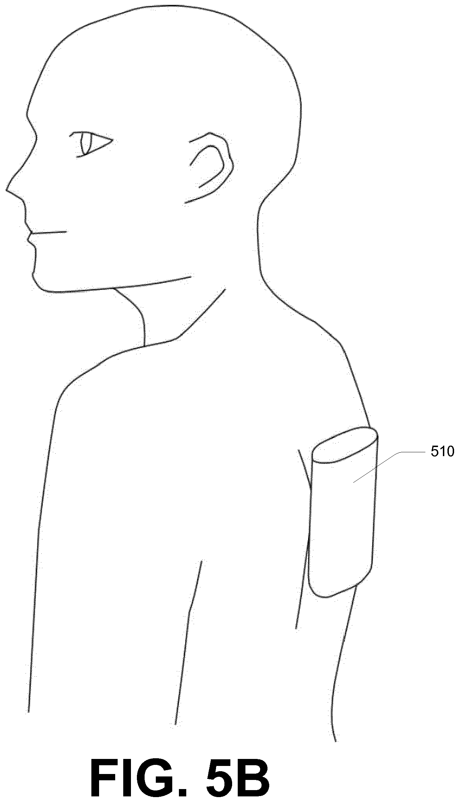

An example secondary body-worn transmitter 110 is illustrated in FIG. 1, showing required hardware components. These are a: a power supply 111, a memory module 112, a processor 113 and a communications module 114 (which may include multiple communication components, thereby to allow transmission/receipt via the primary and secondary wireless communications protocols, for example a BLE module and a WiFi module). It will be appreciated that a wide range of known body-worn devices (or devices able to be presented in a wearable configuration) provide these components. The device may be carried, by way of example, via a helmet (see FIG. 5A) or a back-mounted unit (see FIG. 5B). Other examples include ear-worn devices and skin attached devices (for example as provided by X2 Biosystems).

In the example of FIG. 1, secondary transmitter 110 and a plurality of further mouthguard/body worn transmitter hardware sets 130 are in communication with a computer system via the secondary communications protocol, serves as an impact data processing system 120. System 120 may be defined by substantially any form of computing platform (including an individual terminal or set of networked terminals) having the threshold necessary components of: a power supply 121, memory module 122, processor 123 and communications module 124 configured to receive and transmit over the secondary communications protocol.

System 120 is configured to receive respective tertiary motion data signals from the or each of the one or more human-worn hardware sets, and process the tertiary motion data thereby to determine estimated head impact data for users associated with the or each of the one or more human-worn hardware sets. The nature of this processing varies between embodiments, and depends to a greater extent on the nature of data being collected by the mouthguard device. Specific examples are provided further below in the context of a mouthguard device having seven or more accelerometers (and no gyroscopes).

FIG. 1 also illustrates an example client device 140 having a display screen 141 configured to display data defined by system 120 based on processing of data derived from a given one or more of the mouthguard devices (for example data representative of head impacts, participants who have suffered above threshold head impacts, and so on). For example, in some embodiments device 140 executes a user interface (for example an app or web browser) configured to communicate with system 140 thereby to obtain and present such data. Functions of device 140 are optionally provided by system 130 in further embodiments.

As described above, communications are unidirectional, flowing in a direction from mouthguard 100 to device 140. In some embodiments there is functionality to provide communications in a reverse direction, for example to provide feedback to the participant and/or feedback via the participant that is identifiable to a further person (for example an LED on the mouthguard which could then be seen by another participant, official or coach). In that regard, in some embodiments the secondary transmitter device is configured to receive an input signal via the second wireless communications protocol, and in response cause delivery of an alert signal a wearer of the human worn hardware set. That alert signal may be delivered by the secondary transmitter device, or by the mouthguard. The signal is optionally delivered via one of: haptic feedback; bone conduction (for example using the mouthguard and via teeth); or a visible means.

In the context of defining an upstream alert signal, in some embodiments system 130 is configured to (i) perform a comprehensive impact analysis; and (ii) perform a basic impact analysis and, in response to the basis impact analysis, selectively cause delivery the input signal to the secondary transmitter device. In further embodiments secondary transmitter device performs is configured to process the primary motion data signal thereby to: (i) define the secondary motion data signal, which allows for a comprehensive impact analysis by the computer system; and (ii) perform a basic impact analysis and, in response to the basis impact analysis, selectively cause delivery of an alert signal a wearer of the human worn hardware set.

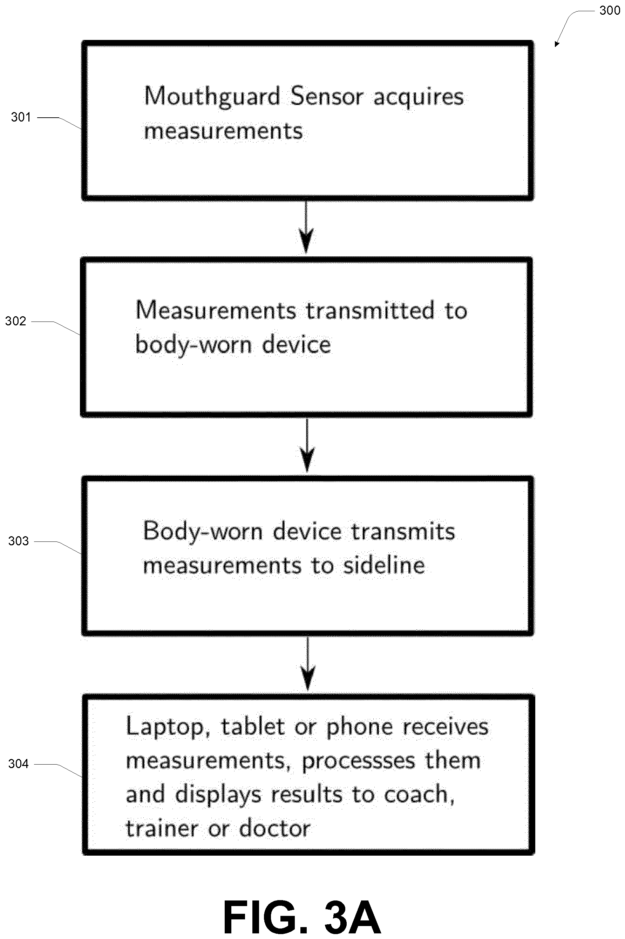

FIG. 3A to FIG. 3D illustrate methods according to various embodiments, which are able to be performed by the framework of FIG. 1 or another similar framework.

Method 300 of FIG. 3A includes: measurements being acquired by a mouthguard sensor device at 301, those measurements being transmitted to a body-worn device at 302, the body worn device transmitting those to a sideline computer system at 303, and a computing device processing the measurements thereby to define result data for transmission to a coach, trainer, doco or the like.

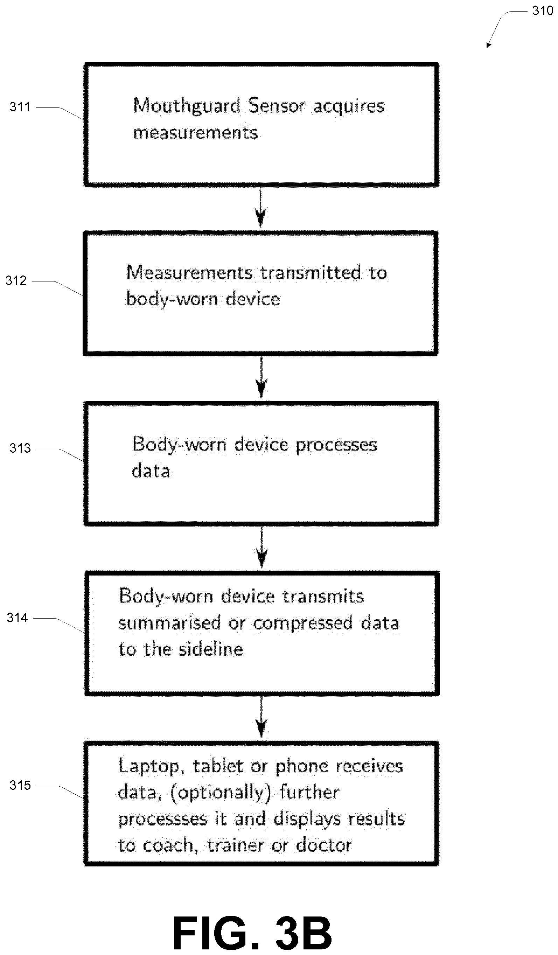

Method 310 of FIG. 3B includes: measurements being acquired by a mouthguard sensor device at 311, those measurements being transmitted to a body-worn device at 312, the body worn device processing received data at 313 thereby to define summary/compressed data, the body worn device transmitting that data to a sideline computer system at 314, and computing device further processing the data (if necessary) and presenting result data for transmission to a coach, trainer, doctor or the like.

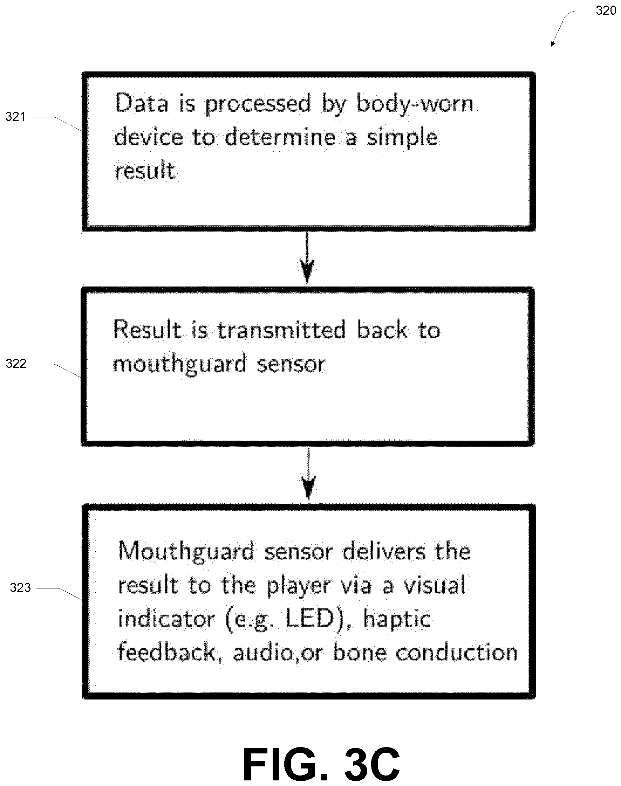

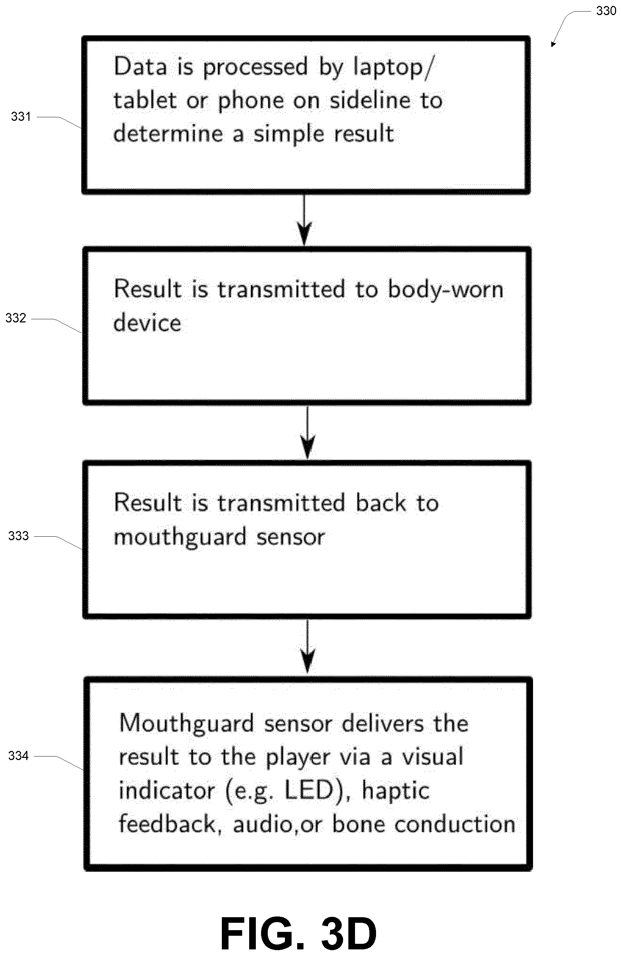

Method 330 of FIG. 3 includes: data being processed by the body-worn device thereby to determine a "simple" result, for example a determination that observed motion data includes above-threshold attributes at 331, result data being transmitted to the mouthguard sensor device at 332, and the mouthguard sensor device delivering feedback representative of the result data at 333. In some cases the result data is limited to an alert signal (indicative of a likely concession or the like); in other embodiments a green light/red light binary notification arrangement is used.

Method 340 of FIG. 3D includes: data being processed by a sideline device thereby to determine a "simple" result, for example a determination that observed motion data includes above-threshold attributes at 341, result data being transmitted to the body-worn device at 342, result data being transmitted to the mouthguard sensor device at 343, and the mouthguard sensor device delivering feedback representative of the result data at 344. Again, in some cases the result data is limited to an alert signal (indicative of a likely concession or the like); in other embodiments a green light/red light binary notification arrangement is used.

It should be appreciated that technology described above enables convenient collection and processing of activity data, thereby to allow reporting on potentially problematic head impacts/injuries.

*Example Mouthguard Device*

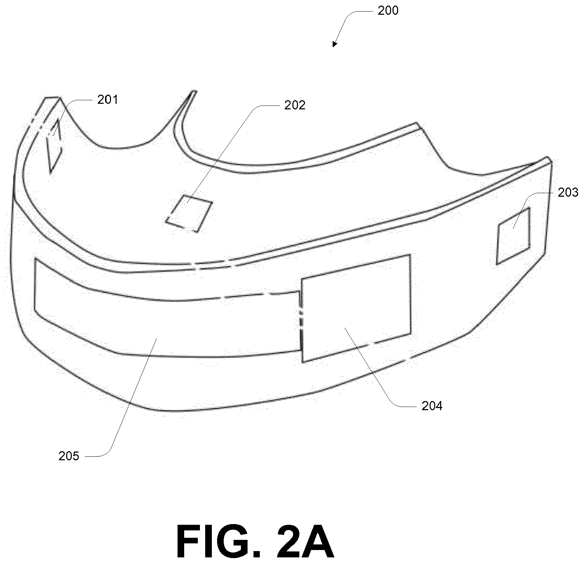

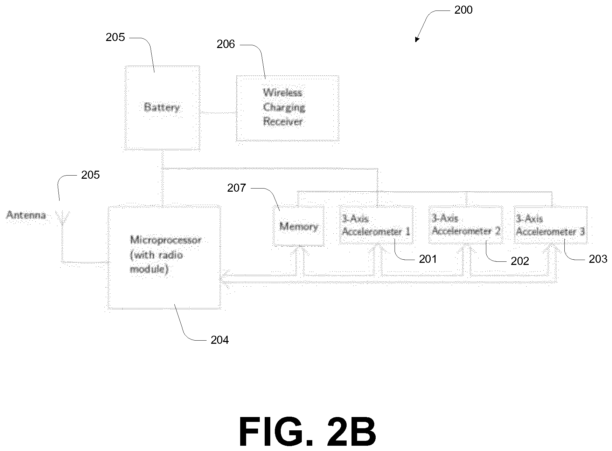

FIG. 2A to FIG. 2C illustrate example mouthguard devices, any of which are optionally used in the context of the framework of FIG. 1. It should be appreciated, however, that the framework of FIG. 1 is not limited in the sense of requiring presence of those particular example mouthguards, and the illustrated mouthguards are also not limited in their application to the framework illustrated in FIG. 1.

A conventional approach for performing motion activity readings is to use a combination of accelerometers and gyroscopes. Accelerometers are useful in measuring linear accelerations; gyroscope readings are differentiated to provide rotational accelerations. It is known to use compact, micro-electromechanical (MEMS) gyroscopes, these having internal vibrating elements configured to measure perturbation of those elements caused by rotation. Unfortunately, these are sensitive to impact, in that impact forces also cause perturbations of the vibrating elements. Thus MEMS gyroscopes are a poor choice for mouthguard impact sensors. Further drawbacks of MEMS gyroscopes include dramatically higher power consumption (compared to accelerometers) and lower bandwidth.

The present inventors have developed technology that allows for accurate determination of linear and rotational accelerations using accelerometers alone (i.e. without the use of gyroscopes), which provides significant advantages in the context of mouthguard sensor devices (for example in terms of size, power efficiency, and overcoming issues noted above in relation to gyroscopes).

As discussed further below, by using particular optimisation methods for sensor data processing, the present inventors have been able to design and functionally configure a mouthguard device having seven or more accelerometers. The mouthguard acts as a device configured to enable analysis of human head motion data. It is defined by a body, forme of resilient plastics material, which is configured to be worn as a mouthguard. For most accurate impact data, and for comfort, the mouthguard should be custom-fitted to the player - not a generic or "boil-and-bite" mouthguard that fits poorly and can move around on the teeth and, hence, give much less accurate data. At least seven accelerometer sensors mounted on the body; these are preferably embedded in cavities within the resilient plastics. The at least seven accelerometer sensors may include one or more 3-axis accelerometer devices. Preferred embodiments described below make use of three 3-axis accelerometer devices, thereby providing nine accelerometer sensors. Although examples are described by reference to such a nine accelerometer implementation, it will be appreciated that the number may be reduced to seven without affecting the ability to use optimisation methods as presently considered. The mouthguard additionally includes a processing device mounted on the body (again preferably embedded within the plastics, for example carried on a circuit board having other components thereon such as a memory module, power supply and the like). The processing device is configured to receive motion data from the at least seven accelerometer sensors.

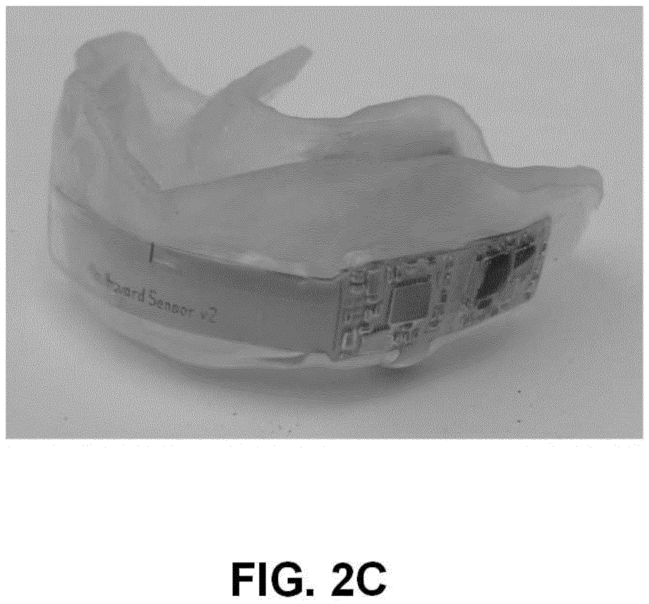

The example of FIG. 1 includes a body 200, with accelerometers 210, 202 and 203 internally mounted at the shown spaced apart locations. A processor circuit board 204 is internally mounted on a frontal region" and this is coupled to an antenna 205 which wraps around a larger fontal region. The antenna is configured to allow wireless communications (for example via BLE as discussed above) for transmission and optionally receipt of digital communications. FIG. 2C provides a schematic illustration having similar reference numerals to those shown in FIG. 2A, additionally separately showing battery 205 and associated wireless charging receiver 206, along with a memory module 207. The example of FIG. 2C illustrates another embodiment of similar configuration to that shown in FIG. 2A.

As described in more detail further below, the received motion data from the at least seven accelerometer sensors is processed thereby to determine values representative of both linear and rotational accelerations of a brain of a wearer of the device. The processing includes applying an optimisation method thereby to, based on a combination of data derived from each of the at least seven accelerometer sensors, determine values representative of both linear and rotational accelerations of a brain of the human head.

*Example Processing Method*

Some embodiments take the form of computer implemented methods configured for receiving motion data derived from at least seven accelerometer sensors, being accelerometer sensors are mounted substantially rigidly to a human head, thereby to determine attributes of head motion. The methods include processing the motion data via an optimisation method thereby to, based on a combination of data derived from each of the at least seven accelerometer sensors, determining values representative of both linear and rotational accelerations of a brain of the human head. Where these methods are used in the context of the framework and mouthguard devices discussed above, the at least seven accelerometer sensors are mounted substantially rigidly to a human head by way of a mouthguard device to which the sensors are mounted.

An example processing method is described in the following sections.

*Rigid Body Motion*

As a general principle, each accelerometer measures the linear acceleration at its centre. Assuming that the accelerometers are rigidly attached to the head/skull, it is possible to model a rigid body motion. We define a (moving) reference frame attached to the head at a point of interest (preferably the centre of the brain). For a rigid body motion of the head, the 3-vector describing the linear acceleration at a point of interest is given by:

$$a_p = a_h + \alpha_h \times r_p + \omega_h \times \left( \omega_h \times r_p \right)$$

where $a_p$ is the acceleration at the point of interest, $a_h$ is the 3-vector linear acceleration of the head and moving reference frame, $\alpha_h$ is the 3-vector rotational acceleration of the head and reference frame, $r_p$ is the 3-vector position of the point of interest with respect to the moving reference frame and $\omega_h$ is the 3-vector rotational velocity of the head and reference frame. For sports impact detection, we can neglect gravitational forces - impacts are much larger.

For a single-axis sensor oriented in an arbitrary direction, we can determine the expected measurement by projecting the 3-vector acceleration onto the desired direction:

$$a_i = \hat{n}_i \cdot \left[ a_h + \alpha_h \times r_i + \omega_h \times \left( \omega_h \times r_i \right) \right]$$

where $a_i$ is the 1-dimensional expected measurement and $\hat{n}_i$ is a unit vector indicating the direction of the sensor.

*Single Time Instant Model*

The vector triple product $\omega_h \times (\omega_h \times r_i)$ may be rewritten as $\omega_i(\omega_i \cdot r_i) - r_i(\omega_i \cdot \omega_i)$. If we rewrite $\omega_h$ as $\omega_{h,perp} + \omega_{h,r}$ where $\omega_{h,perp}$ is the component perpendicular to $r_i$, then we can simplify and re-write the vector triple product as $-r_i(\omega_{h,perp} \cdot \omega_{h,perp})$.

So the acceleration at a point of interest along a particular sensor direction becomes:

$$a_i = \hat{n}_i \cdot \left[ a_h + \alpha_h \times r_i - r_i \left( \omega_{h,perp} \cdot \omega_{h,perp} \right) \right]$$

If we assume that $\omega_{h,perp}$ is approximately the same for all the different accelerometer locations, then for each sensor, the $r_i$ and $\hat{n}_i$ are known and the other values are common across all sensors. Thus, we can write an optimisation problem to solve for the unknown linear and rotational head accelerations and $\omega_{h,perp}$ from at least 7 accelerometer measurements.

For a 9 accelerometer system we can model measured values from the brain centred linear and rotational accelerations as follows:

$$Ax = B + E \qquad (1)$$

where

$$A = \begin{bmatrix} \hat{n}_1 & \hat{n}_1 \cdot [r_1]_x & -\hat{n}_1 \cdot r_1 \\ \hat{n}_2 & \hat{n}_2 \cdot [r_2]_x & -\hat{n}_2 \cdot r_2 \\ \hat{n}_3 & \hat{n}_3 \cdot [r_3]_x & -\hat{n}_3 \cdot r_3 \\ \hat{n}_4 & \hat{n}_4 \cdot [r_4]_x & -\hat{n}_4 \cdot r_4 \\ \hat{n}_5 & \hat{n}_5 \cdot [r_5]_x & -\hat{n}_5 \cdot r_5 \\ \hat{n}_6 & \hat{n}_6 \cdot [r_6]_x & -\hat{n}_6 \cdot r_6 \\ \hat{n}_7 & \hat{n}_7 \cdot [r_7]_x & -\hat{n}_7 \cdot r_7 \\ \hat{n}_8 & \hat{n}_8 \cdot [r_8]_x & -\hat{n}_8 \cdot r_8 \\ \hat{n}_9 & \hat{n}_9 \cdot [r_9]_x & -\hat{n}_9 \cdot r_9 \end{bmatrix}$$

and $x$ is the 7-vector of head linear and rotational accelerations and $\omega_{h,perp}$

$$x = \begin{bmatrix} a_h \\ \alpha_h \\ \omega_{h,perp} \end{bmatrix}$$

and $B$ is the 9x1 measurement vector, consisting of the readings:

$$B = \begin{bmatrix} a_1 \\ a_2 \\ a_3 \\ a_4 \\ a_5 \\ a_6 \\ a_7 \\ a_8 \\ a_9 \end{bmatrix}$$

and $E$ is the measurement error and $[s]_x$ is the skew-symmetric cross product matrix:

$$[s]_x = \begin{bmatrix} 0 & -s_3 & s_2 \\ s_3 & 0 & -s_1 \\ -s_2 & s_1 & 0 \end{bmatrix}$$

Equation (1) is, in preferred embodiments, solved with the pseudo-inverse:

$$\hat{x} = (A^T A)^{-1} A^T B$$

The derivation above assumes a set of 9 acceleration sensors, because accelerometers are readily available in sets of 3 (3-axis accelerometers). A suitably skilled person will recognise that any number of accelerometers can be supported by using the same number of rows in the matrices $A$ and $B$. Note that the vector of parameters $x$ has 7 elements, so a minimum of 7 accelerometers are required.

*Temporal Model Fitting*

The single time instant model above incorporates only measurements (and the sensor noise) from a single time instant. We can improve the estimation by incorporating data from a series of measurements over a period of time. By using many measurements, the sensor noise can be averaged and reduced.

We define a parameterised temporal model for the head linear and rotational accelerations as $a_h(t,X)$ and $\alpha_h(t,X)$ respectively, where $X$ is a vector of parameters and $t$ is the discrete time index. We further model the initial rotational velocity $\omega_{h0}(X)$ and can therefore determine the rotational velocity as a function of time by integration:

$$\omega_h(t,X) = \omega_{h0}(X) + \sum_{s=0}^{t} a_h(s,X)\delta T$$

where $\delta T$ is the sampling period.

For a 1-axis accelerometer, we can model the error as:

$$E_i(t,X) = a_i(t) - \hat{n}_i \cdot [a_h(t,X) + \alpha_h(t,X) \times r_i + \omega_h(t,X) \times (\omega_h(t,X) \times r_i)] \quad (2)$$

Then for a series of time steps and a sequence of measurements, we can compute the total model error as:

$$E(X) = \sum_{t=0}^{N} \sum_{i=1}^{9} E_i(t,X)^2 \qquad (3)$$

We can then apply numerical optimisation methods to find the estimated parameter vector that minimises the model error:

$$\hat{X} = \arg\min_X E(X)$$

Note that this is a non-linear optimisation problem.

Again, the derivation above assumes a set of 9 acceleration sensors. However, a suitably skilled person will recognise that, any number of 7 or more of accelerometers can be utilised and in the accelerometers can be in any distributed arrangement.

*Temporal Models*

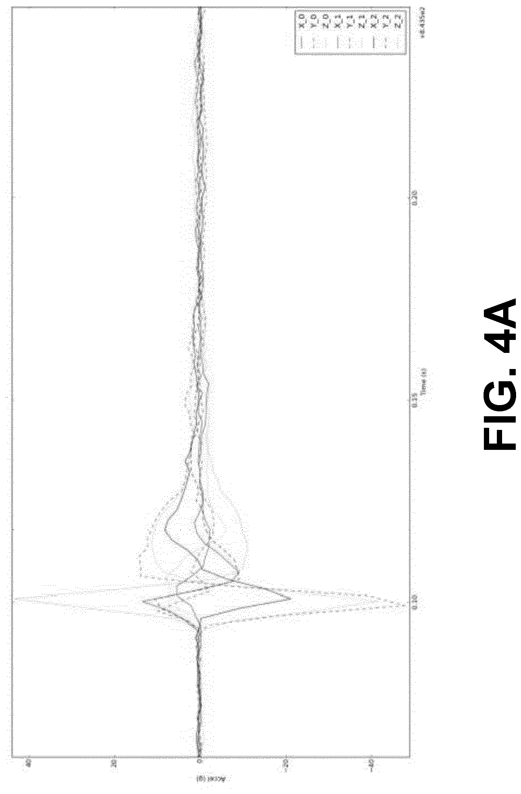

For head impacts, typical curves are as shown FIG. 4A. There is a linear ramp up phase during which the impact is delivered to the head, followed by a damped second-order response as the head rebounds. We define the parameter vector as:

$$X = \begin{bmatrix} t_i \\ t_r \\ a_{max} \\ \alpha_{max} \\ \omega_0 \end{bmatrix}$$

where $t_i$ is the impact time, $t_r$ is the initial rise time, $\alpha_{max}$ is the maximum head linear acceleration, $\alpha_{max}$ is maximum head rotational acceleration and $\omega_0$ is the initial head rotational velocity.

We model the impact activity function as:

$$I(t,X) = \begin{cases} 0 & if \;\; t < t_i - t_r \\ 1 - \dfrac{t_i - t}{t_r} & t < t_i \\ 1 & t = t_r \\ \gamma_1 I(t-1,X) + \gamma_2 I(t-2,X) & otherwise \end{cases}$$

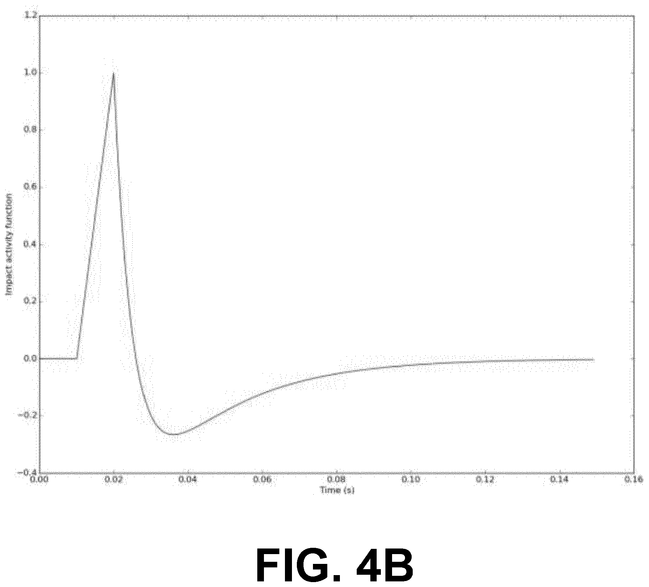

where $\gamma_1$ and $\gamma_2$ are fixed parameters that describe the second-order dynamics of the player's head. The impact activity function is shown in FIG. 4B, showing the initial linear rise and the subsequent damped second-order response.

We then model the head linear acceleration as:

$$a_h(t,X) = a_{max} I(t,X)$$

and the rotational acceleration as:

$$\alpha_h(t,X) = \alpha_{max} I(t,X)$$

This model is a good fit for observed head impact data and contains a minimal number of parameters for fitting head impact data. As such, this model is ideally suited to estimation with head impact data, providing for good, accurate fitting with minimum computational effort. Hence, it is particularly suitable for incorporation into a framework such as that of FIG. 1 above, using mouthguards such as those shown in FIG. 2A to 2C.

A suitably skilled person will recognise that a range of temporal models can be used, with increasing numbers of parameters, up to and including a complete model that includes all of the linear and rotational accelerations at all time instants. However, the best model to use is one that is truly representative of the data in question with the minimum number of parameters.

*Initial Estimate*

The use of pseudo-inverse solving of Equation (1) described above is, in preferred embodiments, used to generate an initial estimate for the parameter vector *X*. This will reduce the time spent performing the numerical optimisation and improve the resultant estimate accuracy.

*Alternate Embodiment #1*

An alternate embodiment is to just use the pseudo-inverse solving of Equation (1) described above to determine the estimated head linear and rotational accelerations at each time step. This approach is much faster and may be suitable for rapid diagnosis or display (for example to provide real-time alert signals as discussed further above). However, the lack of integration over time will yield less accurate results that are more affected by the sensor noise.

*Conclusions and Interpretation*

It will be appreciated that the disclosure above provides various significant systems and methods for monitoring human activity, including novel and innovative technologies relating to mouthguards, data processing, and monitoring communications frameworks.

Unless specifically stated otherwise, as apparent from the following discussions, it is appreciated that throughout the specification discussions utilizing terms such as "processing," "computing," "calculating," "determining", analyzing" or the like, refer to the action and/or processes of a computer or computing system, or similar electronic computing device, that manipulate and/or transform data represented as physical, such as electronic, quantities into other data similarly represented as physical quantities.

In a similar manner, the term "processor" may refer to any device or portion of a device that processes electronic data, e.g., from registers and/or memory to transform that electronic data into other electronic data that, e.g., may be stored in registers and/or memory. A "computer" or a "computing machine" or a "computing platform" may include one or more processors.

The methodologies described herein are, in one embodiment, performable by one or more processors that accept computer-readable (also called machine-readable) code containing a set of instructions that when executed by one or more of the processors carry out at least one of the methods described herein. Any processor capable of executing a set of instructions (sequential or otherwise) that specify actions to be taken are included. Thus, one example is a typical processing system that includes one or more processors. Each processor may include one or more of a CPU, a graphics processing unit, and a programmable DSP unit. The processing system further may include a memory subsystem including main RAM and/or a static RAM, and/or ROM. A bus subsystem may be included for communicating between the components. The processing system further may be a distributed processing system with processors coupled by a network. If the processing system requires a display, such a display may be included, e.g., a liquid crystal display (LCD) or a cathode ray tube (CRT) display. If manual data entry is required, the processing system also includes an input device such as one or more of an alphanumeric input unit such as a keyboard, a pointing control device such as a mouse, and so forth. The term memory unit as used herein, if clear from the context and unless explicitly stated otherwise, also encompasses a storage system such as a disk drive unit. The processing system in some configurations may include a sound output device, and a network interface device. The memory subsystem thus includes a computer-readable carrier medium that carries computer-readable code (e.g., software) including a set of instructions to cause performing, when executed by one or more processors, one of more of the methods described herein. Note that when the method includes several elements, e.g., several steps, no ordering of such elements is implied, unless specifically stated. The software may reside in the hard disk, or may also reside, completely or at least partially, within the RAM and/or within the processor during execution thereof by the computer system. Thus, the memory and the processor also constitute computer-readable carrier medium carrying computer-readable code.

Furthermore, a computer-readable carrier medium may form, or be included in a computer program product.

In alternative embodiments, the one or more processors operate as a standalone device or may be connected, e.g., networked to other processor(s), in a networked deployment, the one or more processors may operate in the capacity of a server or a user machine in server-user network environment, or as a peer machine in a peer-to-peer or distributed network environment. The one or more processors may form a personal computer (PC), a tablet PC, a set-top box (STB), a Personal Digital Assistant (PDA), a cellular telephone, a web appliance, a network router, switch or bridge, or any machine capable of executing a set of instructions (sequential or otherwise) that specify actions to be taken by that machine.

Note that while diagrams only show a single processor and a single memory that carries the computer-readable code, those in the art will understand that many of the components described above are included, but not explicitly shown or described in order not to obscure the inventive aspect. For example, while only a single machine is illustrated, the term "machine" shall also be taken to include any collection of machines that individually or jointly execute a set (or multiple sets) of instructions to perform any one or more of the methodologies discussed herein.

Thus, one embodiment of each of the methods described herein is in the form of a computer-readable carrier medium carrying a set of instructions, e.g., a computer program that is for execution on one or more processors, e.g., one or more processors that are part of web server arrangement. Thus, as will be appreciated by those skilled in the art, embodiments of the present invention may be embodied as a method, an apparatus such as a special purpose apparatus, an apparatus such as a data processing system, or a computer-readable carrier medium, e.g., a computer program product. The computer-readable carrier medium carries computer readable code including a set of instructions that when executed on one or more processors cause the processor or processors to implement a method. Accordingly, aspects of the present invention may take the form of a method, an entirely hardware embodiment, an entirely software embodiment or an embodiment combining software and hardware aspects. Furthermore, the present invention may take the form of carrier medium (e.g., a computer program product on a computer-readable storage medium) carrying computer-readable program code embodied in the medium.

The software may further be transmitted or received over a network via a network interface device. While the carrier medium is shown in an exemplary embodiment to be a single medium, the term "carrier medium" should be taken to include a single medium or multiple media (e.g., a centralized or distributed database, and/or associated caches and servers) that store the one or more sets of instructions. The term "carrier medium" shall also be taken to include any medium that is capable of storing, encoding or carrying a set of instructions for execution by one or more of the processors and that cause the one or more processors to perform any one or more of the methodologies of the present invention. A carrier medium may take many forms, including but not limited to, non-volatile media, volatile media, and transmission media. Non-volatile media includes, for example, optical, magnetic disks, and magneto-optical disks. Volatile media includes dynamic memory, such as main memory. Transmission media includes coaxial cables, copper wire and fiber optics, including the wires that comprise a bus subsystem. Transmission media also may also take the form of acoustic or light waves, such as those generated during radio wave and infrared data communications. For example, the term "carrier medium" shall accordingly be taken to included, but not be limited to, solid-state memories, a computer product embodied in optical and magnetic media; a medium bearing a propagated signal detectable by at least one processor of one or more processors and representing a set of instructions that, when executed, implement a method; and a transmission medium in a network bearing a propagated signal detectable by at least one processor of the one or more processors and representing the set of instructions.

It will be understood that the steps of methods discussed are performed in one embodiment by an appropriate processor (or processors) of a processing (i.e., computer) system executing instructions (computer-readable code) stored in storage. It will also be understood that the invention is not limited to any particular implementation or programming technique and that the invention may be implemented using any appropriate techniques for implementing the functionality described herein. The invention is not limited to any particular programming language or operating system.

It should be appreciated that in the above description of exemplary embodiments of the invention, various features of the invention are sometimes grouped together in a single embodiment, FIG., or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

Similarly, it is to be noticed that the term coupled, when used in the claims, should not be interpreted as being limited to direct connections only. The terms "coupled" and "connected," along with their derivatives, may be used. It should be understood that these terms are not intended as synonyms for each other. Thus, the scope of the expression a device A coupled to a device B should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Thus, while there has been described what are believed to be the preferred embodiments of the invention, those skilled in the art will recognize that other and further modifications may be made thereto without departing from the spirit of the invention, and it is intended to claim all such changes and modifications as falling within the scope of the invention. For example, any formulas given above are merely representative of procedures that may be used. Functionality may be added or deleted from the block diagrams and operations may be interchanged among functional blocks. Steps may be added or deleted to methods described within the scope of the present invention.

**Claims**

1. A system configured to enable analysis of human head impacts, the system including:

   one or more human-worn hardware sets, wherein each human-worn hardware set includes:

   (i) a mouthguard having one or more sensors, wherein the sensors are configured to collectively provide a primary motion data signal, a processor configured to receive the primary motion data signal, and a communications module that is configured to wirelessly transmit, via a first wireless communications protocol, a secondary motion data signal derived from the primary motion data signal;
   (ii) a secondary transmitter device, wherein the secondary transmitter device is configured to receive the secondary motion data signal via the first wireless communications protocol, and in response communicate, via a second wireless communications protocol, a tertiary motion data signal derived from the secondary motion data signal; and

a computer system that is configured to receive respective tertiary motion data signals from the or each of the one or more human-worn hardware sets, wherein the computer system is configured to process tertiary motion data thereby to determine estimated head impact data for users associated with the or each of the one or more human-worn hardware sets.

2. A method according to claim 1 wherein the secondary transmitter device is provided via one of: a helmet; a garment; and another form of body-worn device.

3. A system according to claim 1 wherein the secondary transmitter device is configured to receive an input signal via the second wireless communications protocol.

4. A system according to claim 4 wherein the secondary transmitter device is configured to cause delivery of an alert signal a wearer of the human worn hardware set.

5. A system according to claim 5 wherein the alert signal is delivered by the secondary transmitter device.

6. A system according to claim 5 wherein the alert signal is delivered by the mouthguard.

7. A system according to claim 6 wherein the signal is delivered via one of: haptic feedback; bone conduction; or a visible means.

8. A system according to claim 4 wherein the input signal is defined by the computer system.

9. A system according to claim 9 wherein the computer system is configured to process the tertiary motion data signal thereby to: (i) perform an impact analysis process; and (ii) in response to the impact analysis process, selectively cause delivery the input signal to the secondary transmitter device.

10. A system according to claim 1 wherein the secondary transmitter device performs compression-based processing of the secondary motion data signal as part of defining the tertiary motion data signal.

11. A system according claim 1 wherein the secondary transmitter device performs is configured to process the primary motion data signal thereby to: (i) perform an impact analysis process; and (ii) in response to the impact analysis process,, selectively cause delivery of an alert signal a wearer of the human worn hardware set.

12. A system according to claim 1 wherein at least one of the mouthguards includes at least seven accelerometer sensors mounted on the body; and a processing device mounted on the body, wherein the processing device is configured to receive motion data from the at least seven accelerometer sensors.

13. A system according to claim 12 wherein determine estimated head impact data includes: receiving motion data derived from the least seven accelerometer sensors of a given one of the mouthguards via a tertiary motion data signal; and processing the motion data via an optimisation method thereby to, based on a combination of data derived from each of the at least seven accelerometer sensors, determining values representative of both linear and rotational accelerations of a brain of the human head.

14. A system according to claim 1 wherein at least one of the mouthguards includes one or more of: an accelerometer; a gyroscope; a temperature sensor; a heart rate sensor, a step sensor, and a saliva composition sensor.

15. A system according to claim 1 wherein two or more of the primary motion data signal, secondary motion data signal and tertiary motion data signal include substantially identical data.

**FIG. 1**

# FIG. 2A

# FIG. 2B

# FIG. 2C

300

301 — Mouthguard Sensor acquires measurements

302 — Measurements transmitted to body-worn device

303 — Body-worn device transmits measurements to sideline

304 — Laptop, tablet or phone receives measurements, processses them and displays results to coach, trainer or doctor

# FIG. 3A

310

| 311 | Mouthguard Sensor acquires measurements |

| 312 | Measurements transmitted to body-worn device |

| 313 | Body-worn device processes data |

| 314 | Body-worn device transmits summarised or compressed data to the sideline |

| 315 | Laptop, tablet or phone receives data, (optionally) further processses it and displays results to coach, trainer or doctor |

# FIG. 3B

320

321 — Data is processed by body-worn device to determine a simple result

322 — Result is transmitted back to mouthguard sensor

323 — Mouthguard sensor delivers the result to the player via a visual indicator (e.g. LED), haptic feedback, audio,or bone conduction

# FIG. 3C

330

331

Data is processed by laptop/tablet or phone on sideline to determine a simple result

332

Result is transmitted to body-worn device

333

Result is transmitted back to mouthguard sensor

334

Mouthguard sensor delivers the result to the player via a visual indicator (e.g. LED), haptic feedback, audio,or bone conduction

# FIG. 3D

FIG. 4A

FIG. 4B

500

**FIG. 5A**

510

# FIG. 5B

Europäisches
Patentamt

European
Patent Office

Office européen
des brevets

# EUROPEAN SEARCH REPORT

Application Number

EP 17 20 5498

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/188010 A1 (PARIS ANTHONY J [US] ET AL) 3 July 2014 (2014-07-03) * the whole document * * in particular * * paragraphs 0005, 0006, 0037, 0056-0059; figures 1-3 * | 1-15 | INV. G16H20/30 |
| X | US 2014/187875 A1 (PARIS ANTHONY J [US] ET AL) 3 July 2014 (2014-07-03) * the whole document * * in particular * * paragraphs 0005, 0057-0063, 0065-0068, 0075, 0083-0087; figures 2,3 * | 1-15 | |
| X | US 9 041 528 B2 (HOWARD JOHN W [US]; CUTLER RICHARD [US]; THL HOLDING COMPANY LLC [US]) 26 May 2015 (2015-05-26) * the whole document * * in particular * * abstract; figures 1, 3, 4, 6, 7, 9, , 23, 24, 28, 29-32 * * col 5 lines 1-59; col7 line 17 to col 9 line 58 * | 1-15 | |
| X | US 8 884 756 B2 (HOWARD JOHN W [US]; CUTLER RICHARD [US]; THL HOLDING COMPANY LLC [US]) 11 November 2014 (2014-11-11) * the whole document * | 1-15 | |

TECHNICAL FIELDS SEARCHED (IPC)

G16H
G06F

The present search report has been drawn up for all claims

| Place of search | Date of completion of the search | Examiner |
|---|---|---|
| Munich | 10 April 2018 | Rákossy, Zoltán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 5498

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2018

| Patent document cited in search report | | Publication date | Patent family member(s) | Publication date |
|---|---|---|---|---|
| US 2014188010 | A1 | 03-07-2014 | NONE | |
| US 2014187875 | A1 | 03-07-2014 | NONE | |
| US 9041528 | B2 | 26-05-2015 | NONE | |
| US 8884756 | B2 | 11-11-2014 | NONE | |

EPO FORM P0459

For more details about this annex : see Official Journal of the European Patent Office, No. 12/82

**EP 3 330 971 A1**

**Patent documents cited in the description**

- US 8104324 B
- US 20120143526 A
- US 21000181420 A